# EUROPÄISCHE PATENTANMELDUNG

(11) **EP 1 262 561 A1**
(43) Veröffentlichungstag der Anmeldung: **04.12.2002**
(21) Anmeldenummer: 02010236.4
(22) Anmeldetag: 16.05.2002
(51) Int. Cl.: C12Q 1/68

(54) **Verfahren zum Nachweis und zur Differenzierung von Clavibacter michiganensis**

(30) Priorität: 21.05.2001 DE 10124792
(71) Anmelder: GSF-Forschungszentrum für Umwelt und Gesundheit GmbH, 85764 Oberschleissheim (DE)
(72) Erfinder: Bach, Hans-Jürgen, 80636 München (DE); Jessen, Inga, 85405 Nordstadt (DE); Schloter, Michael, Dr., 85386 Eching (DE); Munch, Jean Charles, Prof. Dr., 73760 Ostfildern (DE)
(74) Vertreter: Maiwald Patentanwalts GmbH

(57) **Zusammenfassung**

Die Erfindung betrifft Oligonukleotide zum qualitativen und quantitativen Nachweis sowie zur Differenzierung von Subspezies der Art *Clavibacter michiganensis*, ein Verfahren zum spezifischen Nachweis und zur Diskriminierung von Subspezies der Art *Clavibacter michiganensis* mittels PCR sowie ein Kit zur Durchführung eines derartigen Verfahrens.

## Beschreibung

Die Erfindung betrifft Oligonukleotide zum qualitativen und quantitativen Nachweis sowie zur Differenzierung von Subspezies der Art *Clavibacter michiganensis,* ein Verfahren zum spezifischen Nachweis und zur Diskriminierung von Subspezies der Art *Clavibacter michiganensis* mittels PCR sowie ein Kit zur Durchführung eines derartigen Verfahrens.

Sämtliche Subspezies der Art *Clavibacter michiganensis* (im folgenden auch mit *C. m.* oder *C. michiganensis* abgekürzt), nämlich *C. m.* subsp. *michiganensis* (*Corynebacterium michiganense*), *C. m.* subsp. *nebraskensis* (*Corynebacterium nebraskense*), *C. m.* subsp. *insidiosus* (*Corynebacterium insidiosum*), *C. m.* subsp. *tessellarius* und *C. m.* subsp. *sepedonicus* (*Corynebacterium sepedonicum*), sind Pflanzenpathogene. Von diesen *Clavibacter michiganensis-Subspezies* ist insbesondere *C. michiganensis* subsp. *sepedonicus* als Erreger der Kartoffelringfäule bekannt. Gemäß Nelson, Can. J. Plant Pathology 1982, 4, 129 - 133 können derartige Infektionen von Setzkartoffeln und Knollen mit *C. m.* subsp. *sepedonicus* auch latent vorliegen, so dass keine äußerlichen Symptome erkennbar sind.

Die anderen vorgenannten Subspezies von *Clavibacter michiganensis* sind ebenfalls pathogen, besitzen allerdings unterschiedliche Wirtspezifitäten (R.R. Carlson und A.K. Vidaver, Int. J. Syst. Bacteriol. 1982, 32, 315 - 326). So verursacht *C. michiganensis* subsp. *michiganensis* Tomatenkrebs (canker on tomato, R. J. Chang et al., Phytopathology 1992, 82, 553 - 560). *C. michiganensis* subsp. *tessellarius* ist der Erreger von bakteriellem Weizenmosaik. Diese Subspezies kann über polyklonale Antikörper (Agdia, Elkhart, Indiana, USA) nachgewiesen werden, die jedoch den Nachteil aufweisen, dass sie mit *C. michiganensis* subsp. *insidiosus* und *C. michiganensis* subsp. *nebraskensis* kreuzreagieren.

*C. michiganensis* subsp. *nebraskensis* verursacht hauptsächlich Laubflecken und Welken bei Mais. Weitere Wirte für diesen Subspezies sind die als Unkräuter bekannten green foxtail (*Setaria viridis*), barnyard grass (*Echinochloa crusgalli*) und shattercane *(Sorghum bicolor*) (M.L. Smidt und A.K. Vidaver, Phytopathology 1987, 77, 388 - 392). *C. michiganensis* subsp. *insidiosus* gilt als verantwortlich für die bakterielle Welke bei Alfalfa und Luzerne (D.A. Samac et al., Plant Dis. 1998, 82, 1362 - 1367).

Die von den vorgenannten Erregern verursachten Pflanzenkrankheiten führen zu erheblichen Ertragseinbußen. Deshalb wurden Maßnahmen gegen die Ausbreitung der Organismen auf den Anbauflächen getroffen. Die Bemühungen konzentrierten sich dabei vor allem auf die Verwendung von reinem Pflanzgut, die Sicherstellung der Erregerfreiheit der für den Anbau vorgesehenen Böden und den Schutz vor Kontakt mit infiziertem Pflanzenmaterial vor allem in Gewächshäusern.

Auf internationaler Ebene wird die Verbreitung dieser Erreger durch strenge Einfuhrbedingungen kontrolliert. Für *C. michiganensis* subsp. *sepedonicus* besteht beispielsweise eine Ein- und Ausfuhrregelung auf Null-Toleranzniveau für die Europäische Union, die USA und Kanada. Herkömmliche Verfahren zum Nachweis der einzelnen *C. michiganensis*-Subspezies basieren auf Enzymimmunoassays (ELISA) (S.H. De Boer et al., Potato research 1992, 35, 207 - 216) und Antikörperreaktionen (N.C. Gudmestad et al., Phytopathology 1991, 81, 475 - 480). Diese bekannten Verfahren sind jedoch auf Grund der großen Ähnlichkeit der Subspezies sehr ungenau. Zusätzlich können auch Kreuzreaktionen mit Organismen anderer Gattungen stattfinden. Bedingt durch die geringe Sensitivität dieser Verfahren müssen ferner teilweise erst Kulturen dieser sehr langsam wachsenden Bakterien angelegt werden.

Auf Grund der vorgenannten Ein- und Ausfuhrregelungen betreffend potentiell mit *C. michiganensis*-Subspezies infiziertem Pflanzenmaterial sowie auf Grund der Tatsache, dass die Organismen auf der Oberfläche von Pflanzenmaterial wie Saat, Blätter oder Früchte, oder in Böden lange unbemerkt existieren können, besteht u.a. seitens der produzierenden Landwirtschaft, von Versicherungen sowie von Pflanzenschutzbehörden ein großer Bedarf an Verfahren, die einen spezifischen, empfindlichen, qualitativen und auch quantitativen Nachweis der Subspezies von *Clavibacter michiganensis* gewährleisten.

Für einen empfindlichen Nachweis bieten sich auf Nukleinsäure-Amplifikation basierende Verfahren an. Es sind bereits mehrere Primer für die spezifische Detektion von *Clavibacter michiganensis* subsp. *sepedonicus* bekannt. So haben Li und De Boer (Can. J. Microbiol. 1995, 41, 925 - 929) Primersequenzen entwickelt, die auf die 16S-23S rDNA Intergenic Spacer-Region gerichtet sind. Mit den dort beschriebenen Primern werden allerdings alle *C. michiganensis*-Subspezies außer *C. michiganensis* subsp. *sepedonicus* diskriminiert, Primer zum Nachweis der anderen *C. michiganensis* Subspezies werden dort nicht erwähnt. Ferner handelt es sich bei dem dort beschriebenen Verfahren lediglich um einen qualitativen Assay.

Lee et al., Appl. Environ. Microbiol. 1997, 63, 2625 - 2630 und 2631 - 2636, haben zwei Sätze von Primern beschrieben, die auf der 16S rDNA bzw. auf der Sequenz eines Insertionselements pCS1, welches sowohl im Chromosom als auch in den Plasmiden vorkommt, basieren. Zum spezifischen Nachweis von *C. michiganensis* subsp. *sepedonicus* is gemäß dieser Veröffentlichung entweder auf 16S rDNA-Ebene eine Nested-PCR (verschachtelte PCR), also zwei hintereinander geschaltete PCR mit unterschiedlichen Primerpaaren, bzw. nach einer Nested-PCR auf pCS1-Ebene noch eine Restriktionsfragment-Längenpolymorphismus-Analyse erforderlich. Eine Quantifizierung ist mit diesem Verfahren nicht möglich.

Firrao und Locci, Can. J. Microbiol. 1994, 40, 148 - 151 haben ebenfalls Primer beschrieben, die auf die vorgenannte Insertionssequenz pCS 1 gerichtet sind. Das Amplikon hat eine Länge von 670 Basenpaaren. Auch mit diesem Verfahren ist die Möglichkeit einer Quantifizierung über PCR nicht gegeben.

Johansen et al. (Phytopathology 1989, 79, 1019 - 1023) haben eine DNA-Sonde beschrieben, die spezifisch an genomische DNA von *C. michiganensis* subsp. *sepedonicus* bindet. Das Detektionslimit für einen Nachweis dieser Subspezies mit der dort beschriebenen DNA-Sond ist allerdings derart hoch, daß der Organismus vor dem Nachweis erst kultiviert werden muss

DNA-Primer für den spezifischen Nachweis der übrigen Subspezies von *C. michiganensis* sind bislang nicht bekannt.

Aufgabe der vorliegenden Erfindung ist es somit, Oligonukleotide bzw. Oligonukleotidsonden bereitzustellen, mit denen die verschiedenen *C. michiganensis*-Subspezies eindeutig nachgewiesen und unterschieden werden können.

Ferner ist es eine Aufgabe der vorliegenden Erfindung, ein Verfahren zur Verfügung zu stellen, mit dem alle *Clavibacter michiganensis*-Subspezies mit hoher Sensitivität spezifisch nachgewiesen sowie differenziert werden können. Weitere Aufgaben ergeben sich aus der folgenden Beschreibung.

Diese Aufgaben werden durch die Gegenstände der unabhängigen Ansprüche gelöst.

Vorteilhafte Ausgestaltungen sind in den Unteransprüchen beschrieben.

Die vorliegende Erfindung basiert auf der Bereitstellung von artspezifischen Primern, mit denen die entsprechenden Gene von allen *C. michiganensis*-Subspezies mittels PCR amplifiziert werden können, sowie auf der Bereitstellung von Oligonukleotidsonden, die eine Unterscheidung zwischen den einzelnen Subspezies gewährleisten.

Die erfindungsgemäßen Oligonukleotidsonden wurden auf Basis von DNA-Sequenzunterschieden im Bereich der 16S-23S rDNA-Spacer-Regionen der fünf *C. michiganensis*-Subspezies derart entwickelt, dass die Sonden jeweils komplementär zu einem Bereich einer bestimmten Subspezies sind, der in den anderen Subspezies in unterschiedlicher Nukleotidzusammensetzung vorliegt.

Insbesondere werden durch die vorliegende Erfindung Oligonukleotidsonden bereitgestellt, die spezifisch an die 16S-23S rDNA Intergenic Spacer-Region von Subspezies der Art *Clavibacter michiganensis* binden, die ausgewählt sind aus der Gruppe, bestehend aus:
a) DNA-Sequenzen mit den in SEQ ID No. 1 bis 5 angegebenen Nukleinsäuresequenzen;
b) DNA-Sequenzen, die zu den in SEQ ID No. 1 bis 5 angegebenen Nukleinsäuresequenzen komplementär sind;
c) DNA-Sequenz, die sich von den in a) bis b) angegebenen Nukleinsäuresequenzen in bis zu drei Nukleotiden unterscheiden;
d) DNA-Sequenzen, die die in a) bis c) angegebenen Nukleinsäuresequenzen oder Fragmente davon umfassen.

Dabei handelt es sich bei der in SEQ ID No. 1 angegebenen DNA-Sequenz um eine für *C. michiganensis* subsp. *sepedonicus* spezifische Sonde, bei der in SEQ ID No. 2 angegebenen Sequenz um eine für *C. michiganensis* subsp. *michiganensis* spezifische Sonde, bei der in SEQ ID No. 3 angegebenen Sequenz um eine für *C. michiganensis* subsp. *nebraskensis* spezifische Sonde, bei der in SEQ ID No. 4 angegebenen Sequenz um eine für *C. michiganensis* subsp. *insidiosus* spezifische Sonde und bei der in SEQ ID No. 5 angegebenen Sequenz um eine für *C. michiganensis* subsp. *tessellarius* spezifische Sonde.

In einer Ausführungsform der Erfindung kann es sich bei den Sonden auch um DNA-Sequenzen handeln, die zu den in SEQ ID No. 1 bis 5 angegebenen Nukleinsäuresequenzen revers komplementär sind.

Unter einer spezifischen Sonde wird im Rahmen der vorliegenden Erfindung ein Oligonukleotid verstanden, welches unter den vorgegebenen Bedingungen nur an eine komplementäre Sequenz bindet und somit alle unterschiedlichen oder nur ähnlichen Sequenzen diskriminiert werden.

Bei einer Nukleinsäuresonde im Sinne der Erfindung kann es sich allgemein um eine DNAoder RNA-gerichtete Sonde handeln, die in der Regel zwischen 12 und 1000 Nukleotide umfassen wird, bevorzugt zwischen 12 und 100 oder 15 und 50 und besonders bevorzugt zwischen 17 und 25 Nukleotide. Vorzugsweise sind die Sonden DNA-Sonden. Alternativ können erfindungsgemäß auch PNA-Sonden eingesetzt werden, bei denen die entsprechender Basen an ein Peptid- bzw. Proteinrückgrat gebunden sind.

Die Nukleinsäuresonde ist so ausgewählt, dass es eine zu ihr komplementäre Sequenz in einem Bereich einer bestimmten *C. michiganensis*-Subspezies gibt, der eine Differenzierung zu den anderen Subspezies zulässt. Komplementarität sollte bei einer Sonde von nur etwa 15 Nukleotiden möglichst über die gesamte Sequenz gegeben sein, während bei Nukleinsäuresequenzen mit mehr als 15 Nukleotiden eine bis mehrere Fehlpaarungsstellen erlaubt sind. Es muss jedoch gewährleistet sein, dass das Nukleinsäuresondenmolekül mit moderaten und/oder stringenten Hybridisierungsbedingungen tatsächlich mit der Zielsequenz hybridisiert bzw. mit zu diskriminierenden Sequenzen nicht hybridisiert.

Unter moderaten und/oder stringenten Hybridisierungsbedingungen werden dabei erfindungsgemäß solche Bedingungen, insbesondere Pufferbedingungen verstanden, wie sie typischerweise in PCR-Reaktionen auftreten. Diese Bedingungen können denen im Folgender angegebenen Beispielen entnommen werden. Dem Fachmann ist dabei bekannt, wie und in welchem Ausmaß er diese Bedingungen variieren kann.

Wird beispielsweise bei der Durchführung des im folgenden beschriebenen Verfahrens zum Nachweis und zur Differenzierung von Subspezies der Art *Clavibacter michiganensis* von einer Annealingtemperatur im Bereich von 62°C bis 68°C, vorzugsweise von 65°C bis 67°C und besonders bevorzugt von 66°C abgewichen, ist eine Differenzierung der Subspezies nicht möglich. So hat eine niedrigere Temperatur zur Folge, dass die eingesetzte Sonde auch an die DNA der anderen Subspezies bindet, während bei einer höheren Temperatur die Sonde an keine der diversen Subspezies-DNA bindet und folglich kein Signal detektiert werden kann.

Die Erfindung betrifft auch Fragmente der in SEQ ID No. 1 bis 5 gezeigten Sequenzen, die eine ausreichende Länge aufweisen, um mit der 16S-23S rDNA Spacer-Region von Subspezies der Art *Clavibacter michiganensis* spezifisch hybridisieren zu können. Die erfindungsgemäßen Oligonukleotidsonden, die einen spezifischen Nachweis und eine spezifische Quantifizierung von Subspezies der Art *Clavibacter michiganensis* ermöglichen, umfassen ferner solche Oligonukleotide, deren Sequenz in einzelnen oder mehreren Nukleotiden durch Addition, Deletion, Substitution oder chemische Modifikationen von den in SEQ ID No. 1 bis 5 gezeigten Sequenzen unmittelbar abgeleiteten Oligonukleotid-Sequenzen abweichen, soweit gleichzeitig eine spezifische Hybridisierung solcher Sonden an die 16S-23S rDNA von Subspezies von *Clavibacter michiganensis* gewährleistet ist.

Ferner wird durch die vorliegende Erfindung ein DNA-Primerpaar bereitgestellt, das spezifisch an Nukleinsäuresequenzen der 16S-23S rDNA Intergenic Spacer Region der Art *Clavibacter michiganensis* bindet. Die erfindungsgemäßen Primer wurden derart ausgewählt, dass sie komplementär zu einem bei allen Subspezies konservierten Bereich sind. Insbesondere werden die erfindungsgemäßen DNA-Primer ausgewählt aus der Gruppe, bestehend aus:
a) DNA-Sequenzen mit den in SEQ ID No. 6 bis 7 angegebenen Nukleinsäuresequenzen;
b) DNA-Sequenzen, die zu den in SEQ ID No. 6 bis 7 angegebenen Nukleinsäuresequenzen komplementär sind;
c) DNA-Sequenz, die sich von den in a) bis b) angegebenen Nukleinsäuresequenzen in bis zu drei Nukleotiden unterscheiden;
d) DNA-Sequenzen, die die in a) bis c) angegebenen Nukleinsäuresequenzen oder Fragmente davon umfassen.

Für die Länge der erfindungsgemäßen DNA-Primer, für die Komplementarität mit dem Zielbereich sowie für Fragmente gilt selbstverständlich dasselbe wie vorstehend für die erfindungsgemäßen Oligonukleotidsonden. Damit umfassen die erfindungsgemäßen DNA-Primer auch die Sequenzen, die revers komplementär zu den in SEQ ID No. 6 bis 7 angegebenen Nukleinsäuresequenzen sind. Bei den DNA-Primern, die zu den in den SEQ ID No. 6 bis 7 angegebenen Nukleinsäuresequenzen (revers) komplementär sind oder Fragmente davon umfassen, kommen nur solche in Frage, mit denen spezifisch ein Teil der 16S-23S rDNA Intergenic Spacer Region amplifiziert werden, d.h. nur solche Primer, die spezifisch mit ihren Zielsequenzen hybridisieren. Bezüglich der Stringenz der Hybridisierungsbedingungen während der PCR gelten im Wesentlichen vergleichbare Bedingungen, wie sie für die oben aufgeführten Oligonukleodtidsonden genannt wurden. Hinweise auf die Bedingungen können den Beispielen entnommen werden.

Ferner können die vorstehend genannten erfindungsgemäßen DNA-Primer als Vorwärtsund/oder Rückwärts-Primer sowie auch als Oligonukleotidsonde im Sinne der vorliegenden Erfindung eingesetzt werden. Ebenso sind die vorstehend genannten erfindungsgemäßen DNA-Sonden dazu geeignet, als DNA-Primer im Sinne der vorliegenden Erfindung zu fungieren, beispielsweise in einem Primerpaar in Kombination mit einem der erfindungsgemäßen DNA-Primer.

Die erfindungsgemäßen Oligonukleotidsonden und DNA-Primer-Sequenzen können sowohl in üblichen Hybridisierungsreaktionen, wie z.B. FISH, Blot-Hybridisierungen und insbesondere Mikroassays, als auch in Amplifikationsverfahren, insbesondere in Polymerase-Ketten-Reaktionen (polymerase chain reaction, PCR) zum spezifischen Nachweis von Subspezies der Art *Clavibacter michiganensis* eingesetzt werden. Dem Fachmann sind die grundlegenden Methoden bekannt; sie sind auch in gängigen molekularbiologischen Laborhandbüchern beschrieben, wie beispielsweise in Sambrook et al. (1989), Molecular Cloning: A laboratory manual, 2. Auflage, Cold Spring Harbour Laboratory Press, Cold Spring Harbour, New York; Griffin und Griffin (1994), PCR-technology - Current innovations, CRC Press, Boca Raton, Ann Arbor, London, Tokyo.

Bei einem wesentlichen Aspekt der vorliegenden Erfindung wird ein Verfahren zum spezifischen Nachweis und zur Differenzierung von Subspezies der Art *Clavibacter michiganensis* mittels PCR bereitgestellt, das folgende Schritte umfasst:
a) artspezifische Amplifikation der 16S-23S rDNA Intergenic Spacer Region der Art *Clavibacter michiganensis* mittels PCR;
b) Inkubation der zu analysierenden DNA mit Oligonukleotidsonden, die spezifisch an die 16S-23S rDNA Intergenic Spacer Region von Subspezies der Art *Clavibacter michiganensis* binden;
c) Detektieren und gegebenenfalls Quantifizieren der hybridisierten Oligonukleotidsonden.

Mit dem erfindungsgemäßen Verfahren können nicht nur alle *Clavibacter michiganensis*-Subspezies über die PCR spezifisch nachgewiesen werden, sondern es kann auch gleichzeitig eindeutig zwischen den verschiedenen Subspezies *C. michiganensis* subsp. *sepedonicus, C. michiganensis* subsp. *nebraskensis, C. michiganensis* subsp. *insidiosus, C. michiganensis* subsp. *tessellarius* sowie *C. michiganensis* subsp. *michiganensis* unterschieden werden.

Bei einer bevorzugten Ausführungsform des erfindungsgemäßen Verfahrens erfolgt der spezifische Nachweis und/oder die Differenzierung von Subspezies der Art *Clavibacter michiganensis* über eine spezifische TaqMan-PCR, die folgende Schritte umfasst:
a) Inkubation der zu analysierenden DNA mit artspezifischen DNA-Primern und für Subspezies spezifischen Oligonukleotidsonden, welche einen gequenchten detektierbaren Marker umfassen;
b) Binden der Primer bzw. der Sonden an die zu analysierende DNA;
c) Primer-Elongation; und
d) Abbau der Sonden durch 5'-Nukleaseaktivität der Taq-Polymerase während der Elongation und Freisetzung von ungequenchtem detektierbarem Marker.

Grundlagen für die Durchführung einer TaqMan-PCR sind in T.A. Schild (1998): Einführung in die Real-Time TaqMan™ PCR-Technologie, Applied Biosystems, angegeben.

Die bei dem erfindungsgemäßen Verfahren verwendeten Oligonukleotide werden vorzugsweise derart ausgewählt, dass die Primer komplementär zu einem bei allen Subspezies konservierten Bereich sind, während die Oligonukleotidsonden jeweils komplementär zu einem differenzierenden Bereich einer bestimmten Subspezies sind.

Ferner ist es bevorzugt, dass die Oligonukleotidsonden möglichst nah an einem der beiden DNA-Primer liegen, da bei der TaqMan-PCR die Sonden effizienter hydrolysiert werden, wenn sie nicht zu weit vom 3'-Ende des Primers entfernt liegen.

Vorzugsweise sind die Zielsequenzen möglichst kurz, vorzugsweise etwa 150 bis 300 Nukleotide, besonders bevorzugt 200 bis 250 Nukleotide und am meisten bevorzugt 223 Nukleotide lang, da die PCR umso effizienter ist, je kürzer die Amplikons sind.

Des weiteren sind bei einer bevorzugten Ausführungsform des erfindungsgemäßen Verfahrens die Annealing-Temperaturen für die Sonden höher als für die DNA-Primer, da so auf jeden Fall gewährleistet ist, dass bei einer erfolgten Bindung der Primer auch die Sonden gebunden haben.

Schließlich ist es bevorzugt, dass die spezifischen Temperaturbedingungen für die Quantifizierung und gleichzeitige Differenzierung bei der PCR für alle fünf *C. michiganensis*-Subspezies gleich sind, so dass bei der Untersuchung einer zu analysierenden Probe ein einheitlicher PCR-Lauf möglich ist.

Dabei kann beispielsweise ein PCR-Lauf mit fünf verschiedenen PCR-Reaktionsmischungen, die jeweils eine für die jeweilige Subspezies von *Clavibacter michiganensis* spezifische Oligonukleotidsonde enthalten, durchgeführt werden. Alternativ ist es ebenfalls möglich, die jeweiligen Oligonukleotidsonden mit unterschiedlichen Markern, beispielsweise Fluoreszenz-Farbstoffen, zu markieren und alle fünf Oligonukleotidsonden gleichzeitig in einer PCR-Reaktionsmischung anzuwenden.

Im Rahmen des erfindungsgemäßen Verfahrens hat ein üblicher PCR-Ansatz bezogen auf 100 µl eine Template-DNA-Konzentration von 5 bis 15 Vol.-% und enthält 10 bis 40 pmol Vorwärts- bzw. Rückwärtsprimer, 5 bis 30 pmol Oligonukleotidsonden, 10 bis 30 nmol Deoxynukleotidtriphosphate und herkömmliche Mengen an Reaktionspuffer und Polymerase sowie sonstige dem Fachmann bekannte Komponenten.

Ein mögliches PCR-Programm bei dem erfindungsgemäßen Verfahren ist wie folgt: 5 bis 15 Min. bei 93°C bis 98°C, 20 bis 60 Zyklen bei 93°C bis 98°C für 10 bis 30 Sek. und 60°C bis 70°C für 30 bis 90 Sek.

Das vorstehend beschriebene Nachweisverfahren basiert auf einem sehr einfachen Analysekit, umfassend lediglich ein Primerpaar sowie gegebenenfalls fünf verschiedene Sonden, und ermöglicht in einem einzigen PCR-Lauf innerhalb kurzer Zeit absolut zuverlässige Aussagen über Qualität und Quantität aller möglichen *C. michiganensis*-Subspezies in einer zu analysierenden Probe.

Bei dem erfindungsgemäßen Verfahren werden die Oligonukleotidsonden vorzugsweise mit Fluoreszenzmarkern eingesetzt, so dass eine einfache Detektion mittels Fluoreszenzmikroskopie möglich ist. Bei einer alternativen Ausführungsform des erfindungsgemäßen Verfahrens erfolgt die Detektion der Oligonukleotidsonden über an die Oligonukleotidsonden gekoppelte detektierbare Marker oder über mit detektierbaren Markern gekoppelte Antikörper. So können die Oligonukleotidsonden durch Biotin- oder Digoxygeninkopplung markiert werden. Die biotinylierten Sonden werden anschließend über Streptavidin-gekoppelte Konjugate, die Digoxygenin-markierten Sonden über Antikörper detektiert.

Weitere mögliche detektierbare Marker werden ausgewählt aus der Gruppe, bestehend aus Chemolumineszenzmarkern, radioaktiven Markern und enzymatischen Gruppen.

Ferner ist es im Rahmen der vorliegenden Erfindung denkbar, die Oligonukleotidsonden zu phosphorylieren bzw. mit Amino- oder Thiolinkern zu versehen.

Vorzugsweise werden bei dem Verfahren als Oligonukleotidsonden die vorstehend beschriebenen erfindungsgemäßen Oligonukleotidsonden verwendet. Ebenso werden bevorzugt als DNA-Primer die vorstehend beschriebenen erfindungsgemäßen DNA-Primer verwendet.

Die Erfindung betrifft des weiteren einen Kit, der für den spezifischen Nachweis und/oder die Differenzierung von Subspezies der Art *Clavibacter michiganensis* mittels PCR eingesetzt werden kann und mindestens eine Oligonukleotidsonde, die spezifisch an die 16S-23S rDNA von Subspezies der Art *Clavibacter michiganensis* bindet, sowie mindestens ein DNA-Primerpaar, das spezifisch an die 16S-23S rDNA Intergenic Spacer Region der Art *Clavibacter michiganensis* bindet, umfasst.

Insbesondere umfasst das erfindungsgemäße Kit mindestens eine vorstehend beschriebene erfindungsgemäße Oligonukleotidsonde und/oder mindestens ein vorstehend beschriebenes erfindungsgemäßes DNA-Primerpaar. Bei einer besonders bevorzugten Ausführungsform umfasst das erfindungsgemäße Kit für jede der Subspezies von *Clavibacter michiganensis* mindestens eine spezifische Oligonukleotidsonde.

Die Oligonukleotidsonden können beispielsweise in lyophylisierter Form oder in einem geeigneten Puffer vorliegen. Weitere Kit-Bestandteile können Enzyme und Reagenzien sein, die üblicherweise bei Hybridisierungs- oder PCR-Reaktionen eingesetzt werden, die ebenfalls in lyophylisierter Form oder in geeigneten Puffern vorliegen. Der Kit kann selbstverständlich auch sämtliche zusätzliche, für das erfindungsgemäße Nachweisverfahren erforderlichen Elemente wie beispielsweise Nukleinsäuresonden zum Durchführen einer Negativkontrolle, umfassen.

Die vorliegende Erfindung betrifft ebenfalls die Verwendung der erfindungsgemäßen Oligonukleotidsonden bzw. DNA-Primer zum spezifischen Nachweis und zur Differenzierung von Subspezies der Art *Clavibacter michiganensis.*

Die nachfolgenden Beispiele dienen der Erläuterung der Erfindung, ohne diese in irgendeiner Weise einzuschränken.

### Beispiel: TaqMan-PCR-Protokoll für die Quantifizierung und Differenzierung der phytopathogenen Clavibacter michiganensis-Subspezies

### a) Organismen und Wachstumsbedingungen

Die in Tabelle 2 aufgeführten untersuchten Organismen wurden von der Deutschen Sammlung für Mikroorganismen und Zellkulturen (DSMZ) bereitgestellt. Die Stämme wurden auf dem empfohlenen Medium bei 30°C unter Schütteln bei 140 rpm in 300 ml gezüchtet. Nachdem ein Wachstum sichtbar war, wurden die Zellsuspensionen durch Zentrifugation bei 4500 x g für eine Dauer von 10 Min. pelletiert. Die Pellets wurden bei -20°C gelagert, bis sie für die Extraktion der genomischen DNA verwendet wurden. Die unterschiedlichen Subspezies von *C. michiganensis* wurden zu Beginn und am Ende der exponentiellen Wachstumsphase für die anschließende Validierung des TaqMan-PCR-Assays geerntet.

### b) Herkömmliche Bakterienmessungen

Die Gesamtzellzahlen wurden nach Membranfiltration und Anfärbung mit 4',6-Diamidino-2-phenylindol (DAPI) (K.G. Porter und Y.S. Feig, Oceanogr. 1980, 25, 943 - 948) wie bei Wagner et al., Appl. Environ. Microbiol. 1993, 59, 1520 - 1525 beschrieben, und durch Zählen von 10 bis 20 unabhängigen Feldern unter Verwendung eines Zeiss-Axioplan-Mikroskops (Zeiss, Oberkochen, Deutschland) bestimmt.

### c) DNA-Extraktion

Die DNA von reinen Kulturen wurde unter Verwendung des DNeasy Tissue Kit (Quiagen, Hilden, Deutschland) extrahiert.

### d) Entwicklung der PCR-Primer und Oligonukleotidsonden

Die 16S-23S rDNA Intergenic Spacer-Sequenzen L43095 (*C. michiganensis* subsp. *tessellarius*), U09378 (*C. michiganensis* subsp. *insidiosus*), U09379 (*C. michiganensis* subsp. *michiganensis*), U09380 (*C. michiganensis* subsp. *michiganensis*), U09381 *(C. michiganensis* subsp. *nebraskensis*) und U09382 (*C. michiganensis* subsp. *sepedonicus*) wurden der NCBI-Datenbank entnommen und in Alignments unter Verwendung des Genomatix DiAlign-Programms (siehe http://genomatix.gsf.de/cgi-bin/dialign/dialign.pl) eingesetzt.

Anschließend wurden homologe Primer-Targetbereiche ausgewählt, die in allen Subspezies konserviert sind und ein DNA-Fragment von 223 Nukleotiden Länge umfassen. Die Sonden-TargetRegionen befinden sich im inneren Teil der Amplikons mit einem Abstand von 81 Nukleotiden zu dem Forward-Primer. Der Vergleich der entwickelten Oligonukleotide mit bekannten DNA-Sequenzen (EMBL, Ausgabe 62) unter Verwendung des Genomatix Matinspector-Programms (siehe http://genomatix.gsf.de/cgi-bin/matinspector/matinspector.pl) ergab, daß das Detektionssystem spezifisch für die für die vorliegende Erfindung relevanten Bakterien ist. Die Oligonukleotide sind in Tabelle 1 angegeben.

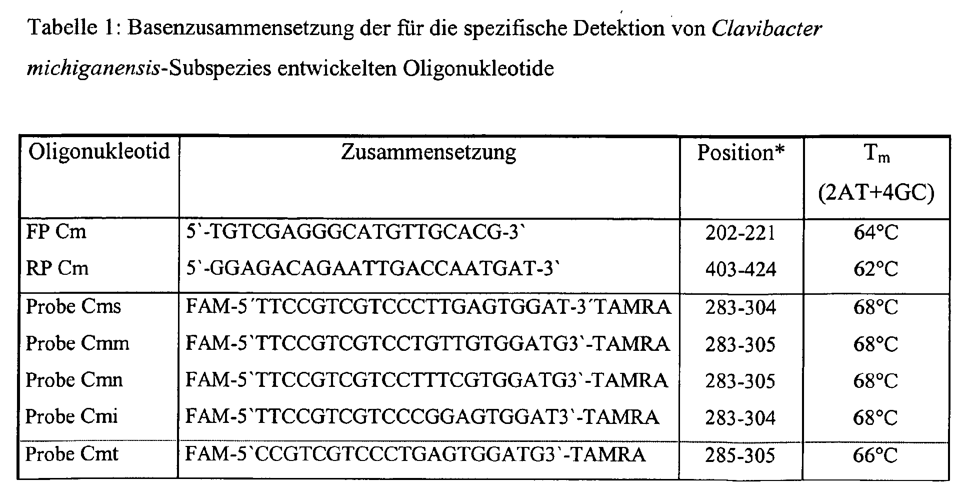

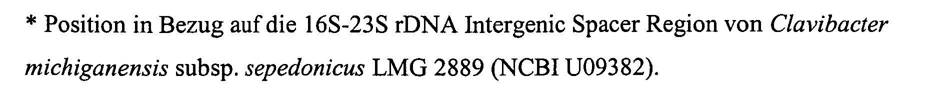

### e) Plasmid-Standards für die absolute Quantifizierung

Die Plasmid-Standards wurden wie folgt hergestellt: Die Intergenic Spacer rDNA-Fragmente der fünf Subspezies wurden durch herkömmliche PCR unter Verwendung der entsprechenden Primer amplifiziert, an welche klebrige Enden an die 5'-Enden angefügt wurden, nämlich die Restriktions-Erkennungssequenz für *Bam*HI plus den Überhang -CGC- an den Forward Primer und die Restriktions-Erkennungssequenz für *Xba*I plus den Überhang -GC- an den Reverse Primer). Die erhaltenen Fragmente wurden verdaut und in den High Copy Number-Vektor Triple Helix™pHelix™ Vektor 1(+) (Roche, Mannheim, Deutschland) kloniert und in *Escherichia coli* JM 105 transformiert.

Die Reinigung der Plasmid-DNA wurde mit dem Plasmid Midi Kit durchgeführt (Qiagen, Hilden, Deutschland). Die Quantität der Plasmid-DNA wurde durch Vergleich der Banden mit jenen des Lambda DNA/*Eco*RI+*Hin*dIII Marker 3 (MBI Fermentas, Vilnius, Litauen) auf Agarosegel nach Ethidiumbromidanfärbung abgeschätzt. Zehnfache Verdünnungen wurden hergestellt und als externe Standards in der TaqMan-PCR verwendet.

### f) PCR

Die Amplifizierung wurde mit dem ABI 7700 Sequence Detection System (Applied Biosystems, Norwalk, Connecticut, USA) durchgeführt. Die Komponenten pro 50 µl waren: 5 µl der Template-DNA, 10 pmol des Forward Primers, 20 pmol des Reverse Primers, 5 pmol der TaqMan-Sonde, 10 nmol Deoxynukleotidtriphosphate, 5 µl von 10 x Reaktionspuffer, 4,0 mM MgCl₂ und 1 U Ampli Taq Gold DNA Polymerase (Applied Biosystems) und H₂O ad 50 µl.

Das PCR-Programm war wie folgt: 10 Min. bei 95°C für die Denaturierung der DNA und die Aktivierung der Polymerase, 40 Zyklen bei 95°C für 20 Sek. und 66°C für 60 Sek. Die qualitative PCR ohne Zugabe von Sonde wurde in 40 µl durchgeführt. Für die quantitative Abschätzung eines jeden Replikats wurden 88 µl Volumeneinheiten hergestellt und in zwei 40 µl-Meß-Replikate aliquotiert, deren Cₜ-Werte gemittelt wurden.

Als Positivkontrollen wurden die PCR zusätzlich mit universellen 16S rDNA gerichteten Primern durchgeführt. Die Komponenten für den 16S rDNA-Assay pro 50 µl waren: 5 µl der Template-DNA, 10 pmol eines jeden Primers, 7,5 pmol der TaqMan-Sonde, 10 nmol Deoxynukleotidtriphosphate, 5 µl von 10 x Reaktionspuffer, 4,5 mM MgCl₂ und 1,25 U Ampli Taq Gold DNA Polymerase (Applied Biosystems) sowie H₂O ad 50 µl.

Das PCR-Programm war wie folgt: 10 Min. bei 95°C für die Denaturierung der DNA und Aktivierung der Polymerase, 40 Zyklen bei 95°C für 20 Sek. und 62°C für 60 Sek.

### g) Ergebnisse

### Spezifität

Zur Bewertung der Spezifität der erfindungsgemäßen Oligonukleotide wurden die unterschiedlichen *C. michiganensis-Subspezies* sowie verschiedene Stämme, die auf dem 16SrDNA-Level nahe verwandt zu *Clavibacter michiganensis* sind, für PCR und TaqMan-PCR getestet. Nahe verwandte Stämme sind solche, die mittels NCBI Blast Search als passend mit der 16S-23S rDNA-Spacer Region von *C. michiganensis* subsp. *sepedonicus* und der 16S rDNA-Sequenz von *C. michiganensis* subsp. *nebraskensis* gefunden wurden. Andere verwandte Organismen sind solche, die in der Literatur zitiert wurden (Li und De Boer, 1995, siehe oben; Lee et al., 1997, siehe oben).

Die Ergebnisse in Tabelle 2 zeigen, daß die *C. michiganensis*-Primer zur Unterscheidung der *C. michiganensis*-Subspezies von nahezu allen anderen Stämmen außer *Rathayibacter* *iranicus,* bei dem eine schwache, aber sichtbare Bande auf Agarosegel nach Durchführung einer 35-Zyklen-PCR sichtbar ist, geeignet ist. Dieser Stamm wird allerdings vollständig in der TaqMan-PCR mit jeder der für *C. michiganensis*-Subspezies spezifischen Sonden diskriminiert.

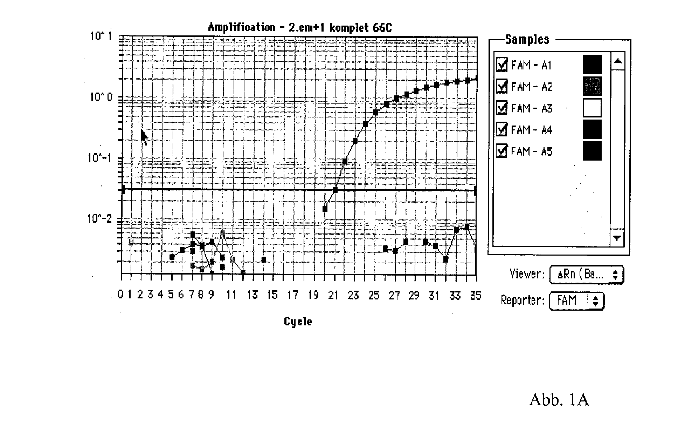

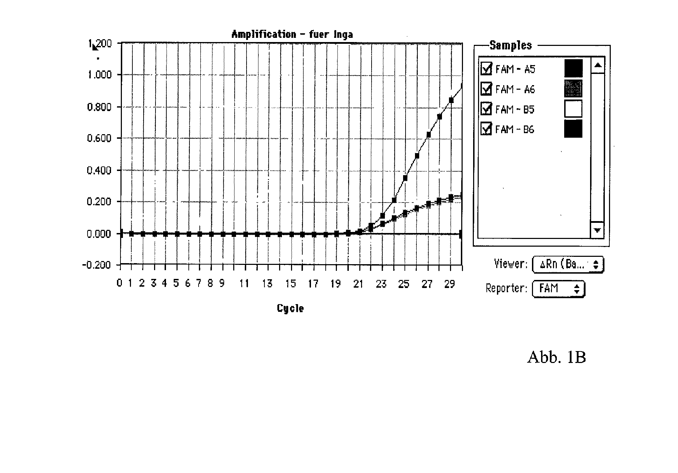

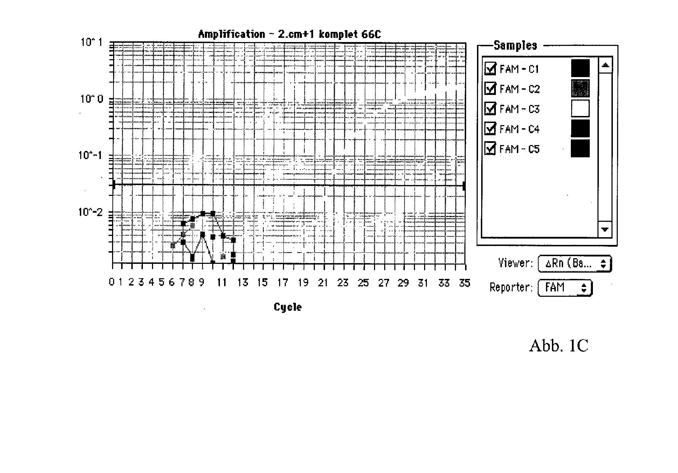

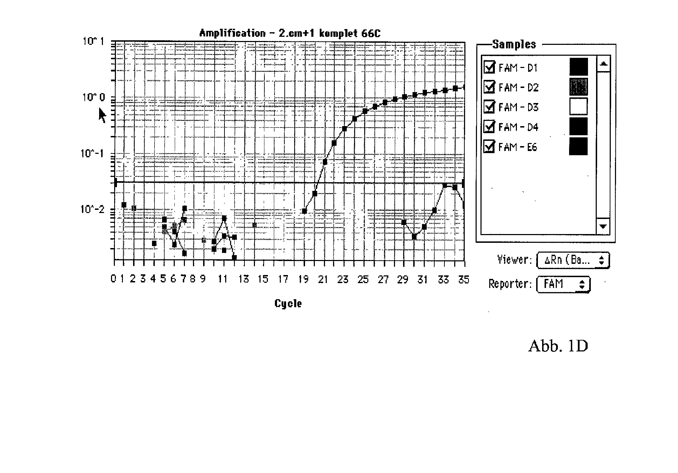

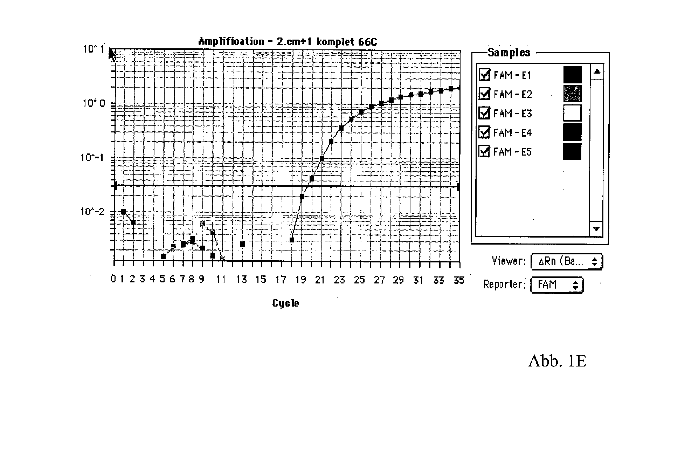

Tabelle 2 zeigt ebenfalls, daß die fluorogenen Sonden hochspezifisch für die Subspezies sind, für die sie entwickelt worden sind. Lediglich die *C. michiganensis* subsp. *tessellarius*-Sonde detektiert schwach die Amplifizierung des *C. michiganensis* subsp. *Sepedonicus*-DNA-Fragments, aber die erzeugte Fluoreszenzkurve ist eindeutig unterscheidbar von der von *C. michiganensis* subsp. *tessellarius* (siehe Abb. 1).

Die Anwendung dieses Assays ist somit geeignet für die zuverlässige Differenzierung unter den Subspezies *C. michiganensis* subsp. *michiganensis, C. michiganensis* subsp. *nebraskensis, C. michiganensis* subsp. *insidiosus, C. michiganensis* subsp. *tessellarius* und *C. michiganensis* subsp. *sepedonicus.*

**Tabelle 2:**

| Spezifität der für die Detektion von *Clavibacter michiganensis*-Subspezies entwickelten Oligonukleotide | | | |
|---|---|---|---|
| Stamm | Amplifikation mit 16S rDNA gerichteten Primern | Amplifikation mit C.m.- Primern | TaqMan-Signal mit Sonden |
| *Clavibacter michiganensis* subsp. *sepedonicus* DSMZ 20744 ^{T} | + | + | C.m.s.-Sonde* |
| *C. m.* subsp. *sepedonicus* DSMZ 1757 | + | + | C.m.s.-Sonde* |
| *C. m.* subsp. *sepedonicus* DSMZ 46300 | + | + | C.m.s.-Sonde* |
| *C. m.* subsp. *sepedonicus* DSMZ 46301 | + | + | C.m.s.-Sonde* |
| *C. m.* subsp. *sepedonicus* DSMZ 46302 | + | + | C.m.s.-Sonde* |
| *C. m.* subsp. *sepedonicus* DSMZ 46304 | + | + | C.m.s.-Sonde* |
| *C. m.* subsp. *michiganensis* DSMZ 46364 ^{T} | + | + | C.m.m.-Sonde |
| *C. m*. subsp. *nebraskensis* DSMZ 7483 ^{T} | + | + | C.m.n.-Sonde |
| *C. m.* subsp. *insidiosus* DSMZ 20157 ^{T} | + | + | C.m.i.-Sonde |
| *C. m.* subsp. *tessellarius* DSMZ 20741 ^{T} | + | + | C.m.t.-Sonde |
| *Clavibacter* sp. DSMZ 6685 | + | - | kein Signal |
| Rathayibacter iranicus DSMZ 7484 ^{T} | + | + | kein Signal |
| *Rathayibacter rathayi* DSMZ 7485 ^{T} | + | | kein Signal |
| *Rathayibacter tritici* DSMZ 7486 ^{T} | + | | kein Signal |
| *Rathayibacter toxicus* DSMZ 7488 ^{T} | + | | kein Signal |
| *Frigoribacter faeni* DSMZ 10309 ^{T} | + | | kein Signal |
| *Cellulomonas biazotea* DSMZ 20112 ^{T} | + | | kein Signal |
| *Cellulomonas fimi* DSMZ 20113 ^{T} | + | | kein Signal |
| *Arthrobacter globiformis* DSMZ 20124 ^{T} | + | | kein Signal |
| *Nocardioides simplex* DSMZ 20130 ^{T} | + | | kein Signal |
| *Nocardioides jensenii* DSMZ 20641 ^{T} | + | | kein Signal |
| *Brevibacter* sp. DSMZ 20419 | + | | kein Signal |
| *Curtobacterium citreum* DSMZ 20528 ^{T} | + | | kein Signal |
| *Curtobacterium luteum* DSMZ 20542 ^{T} | + | | kein Signal |
| *Cellulomonas turbata* DSMZ 20577 ^{T} | + | | kein Signal |
| *Rhodococcus fascians* DSMZ 20669 ^{T} | + | | kein Signal |
| *Streptomyces griseus* subsp. *griseus* DSMZ 40236^{T} | + | | kein Signal |

| | | | |
|---|---|---|---|
| * Schwaches Fluoreszenzsignal wird mit der C.m.t.-Sonde detektiert. | | | |

### Quantifizierung

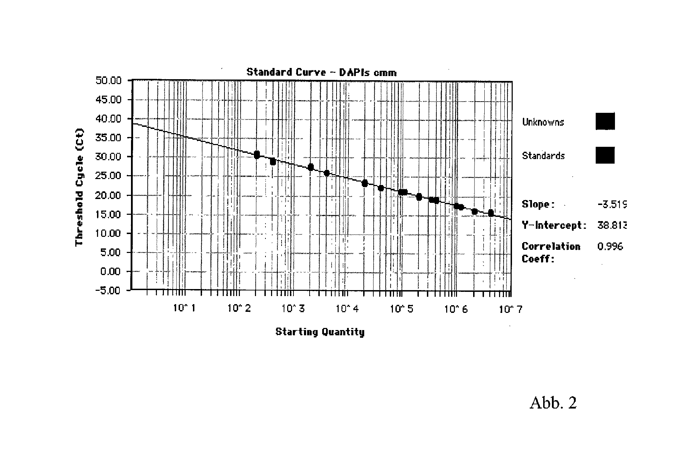

Die durch eine konventionelle PCR mit den *C. michiganensis*-Primern von *C. m.* subsp. *michiganensis, C. m.* subsp. *nebraskensis, C. m.* subsp. *insidiosus, C. m.* subsp. *tessellarius* und *C. m.* subsp. *sepedonicus* erzeugten Amplikons wurden kloniert und als Standard für die Bewertung der Ausgangskopienzahl durch quantitative PCR verwendet. Als Beispiel wird die Standardkurve für *C. michiganensis* subsp. *michiganensis* in Abb. 2 gezeigt. Die Zuverlässigkeit aller fünf TaqMan-PCR-Assays wird durch die in Tabelle 3 gezeigten Standardkurven unterstrichen.

**Tabelle 3:**

| Zuverlässigkeit des TaqMan-PCR-Assays für die unterschiedlichen *C. michiganensis*-Subspezies | | |
|---|---|---|
| TaqMan Quantifizierungs-Assay | Kalibrierungsfunktion | Korrelationskoeffizient |
| *C. michiganensis* subsp. *sepedonicus* | y = -3.56x + 37.76 | 0.996 |
| *C. michiganensis* subsp. *michiganensis* | y = -3.52x + 38.813 | 0.996 |
| *C. michiganensis* subsp. *nebraskensis* | y = -3.73x + 41.59 | 0.987 |
| *C. michiganensis* subsp. *insidiosus* | y = -3.88x + 42.5 | 0.994 |
| *C. michiganensis* subsp. *tessellarius* | y = -3.45x + 37.59 | 0.994 |

Für die Bewertung der Assays wurden die Bakterien in Flüssigkultur gezüchtet und während der stationären Wachstumsphase geerntet. Die Ergebnisse der DAPI-Counts und der Ausgangskopienzahl, die durch TaqMan-PCR bestimmt wurden, zeigen, dass alle fünf Assays geeignet für zuverlässige Messungen der Titer von *C. michiganensis*-Subspezies in DNA-Proben sind (siehe Tabelle 4).

**Tabelle 4:**

| Bewertung der "Real time"-TaqMan-PCR Assays für die Quantifizierung der *Clavibacter michiganensis*-Subspezies in Flüssigkultur. Die Bakterienzellen wurden während der stationären Wachstumsphase geerntet. | | |
|---|---|---|
| Organismus | DAPI-Counts pro ml (Mittelwerte) | Ausgangskopienzahlen pro ml (Mittelwerte) |
| *C. m.* subsp. *sepedonicus* | 8.8 x 10⁴ | 6.2 x 10⁴ |
| *C. m*. subsp. *nebraskensis* | 5.9 x 10⁵ | 10.2 x 10⁵ |
| *C. m*. subsp. *michiganensis* | 7.1 x 10⁵ | 7.0 x 10⁵ |
| *C. m.* subsp. *insidiosus* | 6.0 x 10⁵ | 8.0 x 10⁵ |
| *C. m.* subsp. *tessellarius* | 3.2 x 10⁵ | 3.3 x 10⁵ |

### Abbildungen

Abb. 1: Differenzierung der fünf *C. michiganensis* Subspezies durch spezifische Sonden mittels TaqMan-PCR. Jede Sonde wurde mit fünf unterschiedlichen DNA-Proben, stammend aus *C. michiganensis* subsp. *sepedonicus* (*C.m.s.*), *C. michiganensis* subsp. *tessellarius* (*C.m.t.*), *C. michiganensis* subsp. *nebraskensis* (*C.m.n.*), *C. michiganensis* subsp. *insidiosus* (*C.m.i.*) und *C. michiganensis* subsp. *michiganensis* (*C.m.m.*), untersucht.

A = *C.m.s.*-Sonde als TaqMan-Sonde, B = *C.m.t.*-Sonde als TaqMan-Sonde, C = *C.m.n.*-Sonde als TaqMan-Sonde, D = *C.m.i.*-Sonde als TaqMan-Sonde, E = *C.m.m.*-Sonde als TaqMan-Sonde.

Abb. 2: Eichkurve für die Ausgangskopienzahlen versus Cₜ. Die schwarzen Symbole zeigen die PCR-Amplifizierung der Standard-DNA-Proben und die roten Symbole zeigen die PCR-Amplifizierung von unbekannter genomischer DNA.

## Patentansprüche

1. Oligonukleotide, die spezifisch an die 16S-23S rDNA Intergenic Spacer Region von Subspezies der Art *Clavibacter michiganensis* binden, ausgewählt aus der Gruppe, bestehend aus:
a) DNA-Sequenzen mit den in SEQ ID No. 1 bis 5 angegebenen Nukleinsäuresequenzen;
b) DNA-Sequenzen, die zu den in SEQ ID No. 1 bis 5 angegebenen Nukleinsäuresequenzen komplementär sind;
c) DNA-Sequenz, die sich von den in a) bis b) angegebenen, Nukleinsäuresequenzen in bis zu drei Nukleotiden unterscheiden;
d) DNA-Sequenzen, die die in a) bis c) angegebenen Nukleinsäuresequenzen oder Fragmente davon umfassen.

2. DNA-Primerpaar, das spezifisch an Nukleinsäuresequenzen der Art *Clavibacter michiganensis* bindet,
wobei die DNA-Primer ausgewählt sind aus der Gruppe, bestehend aus:
a) DNA-Sequenzen mit den in SEQ ID No. 6 bis 7 angegebenen Nukleinsäuresequenzen;
b) DNA-Sequenzen, die zu den in SEQ ID No. 6 bis 7 angegebenen Nukleinsäuresequenzen komplementär sind;
c) DNA-Sequenz, die sich von den in a) bis b) angegebenen Nukleinsäuresequenzen in bis zu drei Nukleotiden unterscheiden;
d) DNA-Sequenzen, die die in a) bis c) angegebenen Nukleinsäuresequenzen oder Fragmente davon umfassen.

3. Verfahren zum spezifischen Nachweis und zur Differenzierung von Subspezies der Art *Clavibacter michiganensis* mittels PCR, umfassend folgende Schritte:
a) artspezifische Amplifikation der 16S-23S rDNA Intergenic Spacer Region der Art *Clavibacter michiganensis* mittels PCR;
b) Inkubation der zu analysierenden DNA mit Oligonukleotiden, die spezifisch an die 16S-23S rDNA Intergenic Spacer Region von Subspezies der Art *Clavibacter michiganensis* binden;
c) Detektieren und gegebenenfalls Quantifizieren der hybridisierten Oligonukleotide.

4. Verfahren nach Anspruch 3,
wobei der Nachweis'über eine spezifische TaqMan-PCR erfolgt, die folgende Schritte umfaßt:
a) Inkubation der zu analysierenden DNA mit artspezifischen DNA-Primern und für Subspezies spezifischen Oligonukleotiden, welche einen gequenchten detektierbaren Marker umfassen;
b) Binden der Primer bzw. der Oligonukleotide an die zu analysierende DNA;
c) Primer-Elongation; und
d) Abbau der Oligonukleotide durch 5'-Nukleaseaktivität der Taq-Polymerase während der Elongation und Freisetzung von ungequenchtem detektierbarem Marker.

5. Verfahren nach Anspruch 3 oder 4,
wobei ein Satz von Oligonukleotiden eingesetzt wird, der für jede der Subspezies von *Clavibacter michiganensis* mindestens ein spezifisches Oligonukleotid umfasst.

6. Verfahren nach einem der Ansprüche 3 bis 5,
wobei die Amplikons 150 bis 300 Nukleotide umfassen.

7. Verfahren nach einem der Ansprüche 3 bis 6,
wobei die Annealing-Temperaturen für das Binden der Oligonukleotidsonden höher sind als für das Binden der DNA-Primer.

8. Verfahren nach einem der Ansprüche 3 bis 7,
wobei die Temperaturbedingungen für die Quantifizierung und Differenzierung für alle eingesetzten Oligonukleotidsonden gleich sind.

9. Verfahren nach einem der Ansprüche 3 bis 8,
wobei die Detektion der Oligonukleotide über an die Oligonukleotide gekoppelte detektierbare Marker oder über mit detektierbaren Markern gekoppelte Antikörper erfolgt.

10. Verfahren nach einem der Ansprüche 3 bis 9, wobei der detektierbare Marker aus der Gruppe, bestehend aus Fluoreszenzmarkern, Chemolumineszenzmarkern, radioaktiven Markern und enzymatischen Gruppen, ausgewählt wird.

11. Verfahren nach einem der Ansprüche 3 bis 10, wobei als Oligonukleotide die Oligonukleotide nach Anspruch 1 verwendet werden.

12. Verfahren nach einem der Ansprüche 3 bis 11, wobei als DNA-Primer die DNA-Primer nach Anspruch 2 verwendet werden.

13. Kit zur Durchführung des Verfahrens nach einem der Ansprüche 3 bis 12, umfassend:
a) mindestens ein Oligonukleotid, das spezifisch an die 16S-23S rDNA Intergenic Spacer Region von Subspezies der Art *Clavibacter michiganensis* bindet,
b) mindestens ein DNA-Primerpaar, das spezifisch an Nukleinsäuresequenzen der 16S-23S rDNA Intergenic Spacer Region der Art *Clavibacter michiganensis* bindet.

14. Kit nach Anspruch 13, umfassend mindestens ein Oligonukleotid nach Anspruch 1 sowie mindestens ein DNA-Primerpaar nach Anspruch 2.

15. Kit nach Anspruch 13 oder 14, der für jede der Subspezies von *Clavibacter michiganensis* mindestens ein spezifisches Oligonukleotid umfasst.

16. Kit nach einem der Ansprüche 13 bis 15, ferner umfassend mindestens eine Nukleinsäuresonde zum Durchführen einer Negativkontrolle.

17. Kit nach einem der Ansprüche 13 bis 16, ferner umfassend einen Hybridisierungspuffer.

18. Verwendung eines Oligonukleotids nach Anspruch 1 und/oder eines DNA-Primerpaars nach Anspruch 2 zum spezifischen Nachweis und zur Differenzierung von Subspezies der Art *Clavibacter michiganensis.*

19. Verwendung nach Anspruch 18 in einem Verfahren gemäß einem der Ansprüche 3 bis 12.


SEQUENZPROTOKOLL

<110> GSF - Forschungszentrum für Umwelt und Gesundheit

<120> Verfahren zum Nachweis und zur Differenzierung von
      pflanzenpathogenen Subspezies der Art Clavibacter
      michiganensis

<130> G 7221

<140> DE 101 24 792
<141> 2001-05-21

<160> 7

<170> PatentIn Ver. 2.1

<210> 1
<211> 22
<212> DNA
<213> Künstliche Sequenz

<220>
<223> Beschreibung der künstlichen Sequenz:
      Oligonukleotidsonde

<400> 1
ttccgtcgtc ccttgagtgg at                                          22

<210> 2
<211> 22
<212> DNA
<213> Künstliche Sequenz

<220>
<223> Beschreibung der künstlichen Sequenz:
      Oligonukleotidsonde

<400> 2
ttccgtcgtc ctgttgtgga tg                                          22

<210> 3
<211> 22
<212> DNA
<213> Künstliche Sequenz

<220>
<223> Beschreibung der künstlichen Sequenz:
      Oligonukleotidsonde

<400> 3
ttccgtcgtc ctttcgtgga tg                                          22

<210> 4
<211> 21
<212> DNA
<213> Künstliche Sequenz

<220>
<223> Beschreibung der künstlichen Sequenz:

Oligonukleotidsonde

<400> 4
ttccgtcgtc ccggagtgga t                                                21


<210> 5
<211> 20
<212> DNA
<213> Künstliche Sequenz

<220>
<223> Beschreibung der künstlichen Sequenz:
      Oligonukleotidsonde

<400> 5
ccgtcgtccc tgagtggatg                                                  20


<210> 6
<211> 20
<212> DNA
<213> Künstliche Sequenz

<220>
<223> Beschreibung der künstlichen Sequenz:
      Oligonukleotidprimer

<400> 6
tgtcgagggc atgttgcacg                                                  20


<210> 7
<211> 22
<212> DNA
<213> Künstliche Sequenz

<220>
<223> Beschreibung der künstlichen Sequenz:
      Oligonukleotidprimer

<400> 7
ggagacagaa ttgaccaatg at                                               22